# FASCICULE DE BREVET EUROPEEN

(11) **EP 3 552 575 B1**
(45) Date de publication et mention de la délivrance du brevet: **10.03.2021**
(21) Numéro de dépôt: 18305458.4
(22) Date de dépôt: 13.04.2018
(51) Int. Cl.: A61C 9/00, A61B 1/24, G16H 30/20, G16H 50/00, A61B 5/00

(54) **PROCÉDÉ DE GÉNÉRATION D'UN MODÈLE 3D D'UNE ARCADE DENTAIRE**
VERFAHREN ZUR ERZEUGUNG EINES 3D-MODELLS EINER ZAHNREIHE
METHOD FOR GENERATING A 3D MODEL OF A DENTAL ARCH

(43) Date de publication de la demande: 16.10.2019
(73) Titulaire: Dental Monitoring, 75008 Paris (FR)
(72) Inventeur: SALAH, Philippe, 93170 BAGNOLET (FR); GHYSELINCK, Guillaume, 59169 CANTIN (FR); PELLISSARD, Thomas, 92110 CLICHY (FR); DEBRAUX, Laurent, 75020 PARIS (FR)
(74) Mandataire: Nony

(56) Documents cités:
- EP-A2- 1 252 858
- US-A1- 2003 012 423
- US-A1- 2017 128 173
- I. AHMAD: "Digital dental photography. Part 8: intra-oral set-ups", BDJ, vol. 207, no. 4, 22 août 2009 (2009-08-22) , pages 151-157, XP055209322, ISSN: 0007-0610, DOI: 10.1038/sj.bdj.2009.715

## Description

### Domaine technique

La présente invention concerne un procédé de génération d'un modèle numérique tridimensionnel d'une arcade dentaire d'un patient, et un dispositif de prise de vue dentaire pouvant être mis en œuvre dans le cadre de ce procédé.

### Etat de la technique

La fabrication des appareils orthodontiques repose de plus en plus sur des modèles tridimensionnels numériques des arcades du patient. Ces modèles sont classiquement obtenus au moyen d'un scanner optique intra-oral avec lequel l'orthodontiste balaie l'arcade du patient.

Cette opération est coûteuse et nécessite un déplacement du patient chez l'orthodontiste. En outre, la réalisation d'un scan intra-oral est désagréable, voire douloureuse, et longue à réaliser.

US 2003/0012423 décrit un procédé de génération d'un modèle d'une dent à partir d'images, mais requiert la mise en œuvre d'une cible, par exemple d'une agrafe rigide.

Il existe donc un besoin pour un nouveau procédé permettant de générer un modèle numérique tridimensionnel d'une arcade dentaire d'un patient et qui ne présente pas les inconvénients susmentionnés.

Un objectif de la présente invention est de répondre, au moins partiellement, à ce besoin.

### Résumé de l'invention

L'invention concerne un procédé de génération d'un modèle numérique tridimensionnel d'une arcade dentaire d'un patient selon la revendication 1.

Par « simultanément à l'étape A) », on entend que la série d'images actualisées est acquise alors que le faisceau est projeté. La projection peut cependant commencer avant et se prolonger après l'acquisition des images actualisées.

Un procédé selon l'invention présente de préférence une ou plusieurs des caractéristiques optionnelles suivantes :
- à l'étape C), pour fabriquer le modèle actualisé, on modifie un modèle défini en fonction de caractéristiques du patient ;
- à l'étape C), on détermine le modèle actualisé comme étant le modèle à tester obtenu à la fin du cycle d'étapes a) à c) suivant :
   a) création d'un modèle à tester, puis
   b) détermination d'une distance représentative de la différence entre l'ensemble des projections actualisées et le modèle à tester, puis,
   c) si ladite distance représentative dépasse un seuil d'acceptabilité prédéterminé, modification du modèle à tester et reprise à l'étape a) ;
- on évalue la distance représentative à partir de distances élémentaires, chaque distance élémentaire étant déterminée, pour une projection actualisée respective, par une évaluation de la différence entre ladite projection actualisée et une projection de référence optimale,
   une projection de référence étant une représentation, sur une image de référence représentant une vue du modèle à tester, d'une marque lumineuse virtuelle résultant de la projection, sur le modèle à tester, d'un faisceau lumineux virtuel de même forme que le faisceau lumineux projeté sur l'arcade à l'étape A),
   l'image de référence optimale étant l'image de référence faisant apparaître la projection de référence qui présente une distance minimale avec la projection actualisée ;
- le modèle à tester est segmenté de manière à définir des modèles de dent et la modification du modèle à tester comprend des déplacements des modèles de dent et/ou des déformations de ces modèles de dent ;
- le premier modèle à tester à être modifié est un modèle d'arcade dentaire, de préférence sélectionné en fonction de caractéristiques du patient ;
- à l'étape C), on met en œuvre au moins deux méthodes parmi les méthodes d'optimisation, les méthodes d'intelligence artificielle, les méthodes d'évaluation de dimensions par stéréovision, les méthodes d'évaluation de dimensions par analyse de la forme de la marque lumineuse et les méthodes d'évaluation de dimensions par analyse de la distance entre des points remarquable des images actualisées ;
- à l'étape A), on projette un faisceau de lumière structurée ;
- à l'étape B), le patient porte un écarteur dentaire immobilisé par rapport à un appareil d'acquisition desdites images actualisées ;
- à l'étape B), le patient déplace ladite arcade par rapport audit appareil d'acquisition d'images.

Le procédé selon l'invention met en oeuvre un dispositif d'acquisition comportant :
- un support ;
- un écarteur dentaire fixé sur le support et définissant une ouverture d'écarteur ;
- un appareil d'acquisition d'images actualisées fixé sur le support dans une position dans laquelle il observe l'ouverture d'écarteur suivant un axe optique.

**Suivant un premier aspect principal,** le dispositif comporte encore
- un projecteur adapté pour projeter un faisceau lumineux vers l'ouverture d'écarteur, de manière à dessiner, lorsque l'écarteur est porté par un patient, au moins une marque lumineuse sur une arcade du patient, ladite marque lumineuse étant représentée sur chaque image actualisée par une projection actualisée respective, et
- un module de traitement, de préférence intégré sur le support, configuré pour identifier les projections actualisées sur les images actualisées et pour fabriquer un modèle actualisé présentant une concordance maximale avec l'ensemble des projections actualisées identifiées.

Après mise en place de l'écarteur sur la bouche du patient, l'appareil d'acquisition d'images peut ainsi acquérir une série d'images actualisées de l'arcade faisant chacune apparaître une représentation de la marque résultant de la projection du faisceau sur l'arcade, puis fabriquer un modèle actualisé correspondant.

Le dispositif est avantageusement peu coûteux, léger et portatif, et peut être mis en œuvre facilement par le patient lui-même.

Plus généralement, le dispositif est configuré pour mettre en œuvre l'étape A), l'acquisition des images actualisées à l'étape B) et l'étape C).

**Suivant un deuxième aspect principal,** le dispositif comporte un télémètre disposé de manière à mesurer une distance entre un point du support, par exemple un point de l'appareil d'acquisition d'images, et un objet obturant l'ouverture d'écarteur, par exemple une arcade dentaire du patient portant l'écarteur.

Le télémètre est de préférence en communication avec l'appareil d'acquisition d'images. Le module de traitement est de préférence configuré pour calibrer l'appareil d'acquisition d'images en fonction d'une mesure reçue du télémètre.

Le télémètre permet de déterminer avec précision la distance entre l'arcade et l'appareil d'acquisition d'images, ce qui permet de parfaitement calibrer l'appareil d'acquisition d'images et facilite encore la recherche du modèle actualisé.

Le dispositif mis en oeuvre dans le procédé selon l'invention présente de préférence une ou plusieurs des caractéristiques optionnelles suivantes :
- le faisceau est plan et présente une épaisseur inférieure à 1 mm et une largeur supérieure à 1 cm lorsqu'il traverse l'ouverture d'écarteur ;
- l'appareil d'acquisition d'images et/ou le module de traitement sont intégrés sur le support ;
- le projecteur est configuré pour projeter un faisceau non visible à l'œil nu ;
- le projecteur est configuré pour projeter un faisceau ultraviolet ;
- l'appareil d'acquisition d'images comporte un capteur multispectral ;
- le support et/ou l'écarteur comportent un miroir configuré pour réfléchir une image vers l'appareil d'acquisition d'images et/ou un miroir configuré pour réfléchir le faisceau lumineux, et/ou une mire colorimétrique et/ou une mire de translucidité ;
- le module de traitement est configuré pour calibrer l'appareil d'acquisition d'images en fonction d'une observation de la mire colorimétrique et/ou de la mire de translucidité ;
- le support et/ou l'écarteur comportent une caméra thermique et/ou une sonde de température et/ou un analyseur d'haleine ;
- l'appareil d'acquisition d'images est à une distance du centre de l'ouverture d'écarteur inférieure à 20 cm, voire inférieure à 10 cm ;
- le projecteur est configuré de manière à faire varier la longueur d'onde du faisceau au cours du temps ;
- le dispositif comporte une source lumineuse diffuse ;
- la source lumineuse présente de préférence un indice de rendu des couleurs supérieur à 80 ;
- l'appareil d'acquisition d'images est configuré pour commander la source lumineuse.

Sont décrits également :
- un programme d'ordinateur comprenant des instructions de code de programme pour mettre en œuvre une étape C) et, de préférence commander l'appareil d'acquisition d'images et/ou le projecteur lors d'une étape B),
- un support informatique sur lequel est enregistré un tel programme, par exemple une mémoire ou un CD-ROM, et
- un module de traitement d'images dans lequel est chargé un tel programme.

### Définitions

Par « patient », on entend toute personne pour laquelle un procédé selon l'invention peut être mis en œuvre, que cette personne soit malade ou non, ou que cette personne soit en cours de traitement ou non.

Par « marque lumineuse », on entend le résultat de l'interaction entre un faisceau lumineux et une arcade dentaire. Une marque lumineuse peut être un point, une ligne, une bande ou un ensemble de points et/ou de lignes et/ou de bandes. Une ligne peut donc être continue ou localement interrompue et constituée de morceaux de lignes. Elle peut présenter une largeur constante ou variable. La représentation d'une marque lumineuse, en particulier sur une image actualisée, varie en fonction de la direction d'observation de cette marque.

Le qualificatif « lumineux » inclut toutes les ondes électromagnétiques depuis l'infrarouge jusqu'à l'ultraviolet.

Par « modèle », on entend une représentation numérique en trois dimensions, ou « modèle 3D ».

Par "image", on entend une représentation numérique en deux dimensions, comme une photographie ou une image extraite d'un film. Une image est formée de pixels.

Par « image d'une arcade » ou « modèle d'une arcade », on entend une représentation, en deux ou trois dimensions, respectivement, de tout ou partie de ladite arcade.

Une image de référence est une image obtenue par observation d'un modèle suivant une direction d'observation de référence.

Dans l'environnement virtuel d'un modèle d'une arcade, un faisceau virtuel de même forme que le faisceau projeté sur l'arcade du patient, ou « faisceau actualisé », peut être projeté sur le modèle de manière à obtenir une marque lumineuse dite « virtuelle ». La marque lumineuse virtuelle peut être observée suivant différents angles, chaque vue constituant une image de référence. La représentation de la marque lumineuse virtuelle sur une image de référence est dite « projection de référence ».

Un modèle historique est un modèle d'arcade d'une base d'apprentissage destinée à l'entrainement d'un réseau de neurones. La projection, sur un modèle historique, d'un faisceau historique de même forme que le faisceau projeté sur l'arcade du patient produit une marque lumineuse dite « historique ». La marque lumineuse historique peut être observée suivant différents angles, chaque vue constituant une image historique. La représentation de la marque lumineuse historique sur une image historique est dite « projection historique ».

Une base d'apprentissage comporte des modèles historiques, de préférence plus de 1000, de préférence plus de 10 000, de préférence plus de 100 000 modèles historiques, et, pour chaque modèle historique, associe un ensemble de projections historiques.

Une représentation de la marque lumineuse sur une image actualisée est dite « projection actualisée ».

On appelle « concordance » (« *match »* ou *« fit »* en anglais) entre deux représentations d'un objet une mesure de la différence, ou « distance », entre ces deux objets. Une concordance est maximale (« *best fit* ») lorsque cette différence est minimale.

Deux images ou « vues » qui présentent une concordance maximale représentent idéalement sensiblement un même objet, de la même façon. Autrement dit, les représentations de l'objet sur ces deux images sont sensiblement superposables.

Une projection actualisée est en concordance maximale avec une projection de référence identifiée sur une image de référence lorsque cette image de référence est, parmi l'ensemble des images de référence observables sur le modèle, celle qui présente la projection de référence écartée de la projection actualisée par une « distance » minimale. Cette projection de référence est dite « optimale ».

Un modèle est en concordance maximale avec un ensemble de projections actualisées lorsqu'une distance représentative de la différence entre l'ensemble des projections actualisées et le modèle est minimale.

Par « intégré au support », on entend « fixé de manière non amovible sur le support ».

"Comprendre", "comporter" et "présenter" doivent être interprétés de manière large et non limitative, sauf indication contraire.

### Brève description des figures

D'autres caractéristiques et avantages de l'invention apparaîtront encore à la lecture de la description détaillée qui va suivre et à l'examen du dessin annexé dans lequel :
- la figure 1 représente, schématiquement, une coupe verticale longitudinale d'un dispositif selon l'invention ;
- la figure 2 représente, vu de dessus, le dispositif de la figure 1 ;
- la figure 3 représente un exemple de modèle actualisé ;
- la figure 4 représente un écarteur conventionnel ;
- la figure 5 représente schématiquement un exemple de faisceau ;
- la figure 6 illustre schématiquement un procédé selon l'invention ; et
- la figure 7 représente une marque lumineuse, en l'occurrence une grille lumineuse, projetée sur une arcade dentaire.

### Description détaillée

### Dispositif

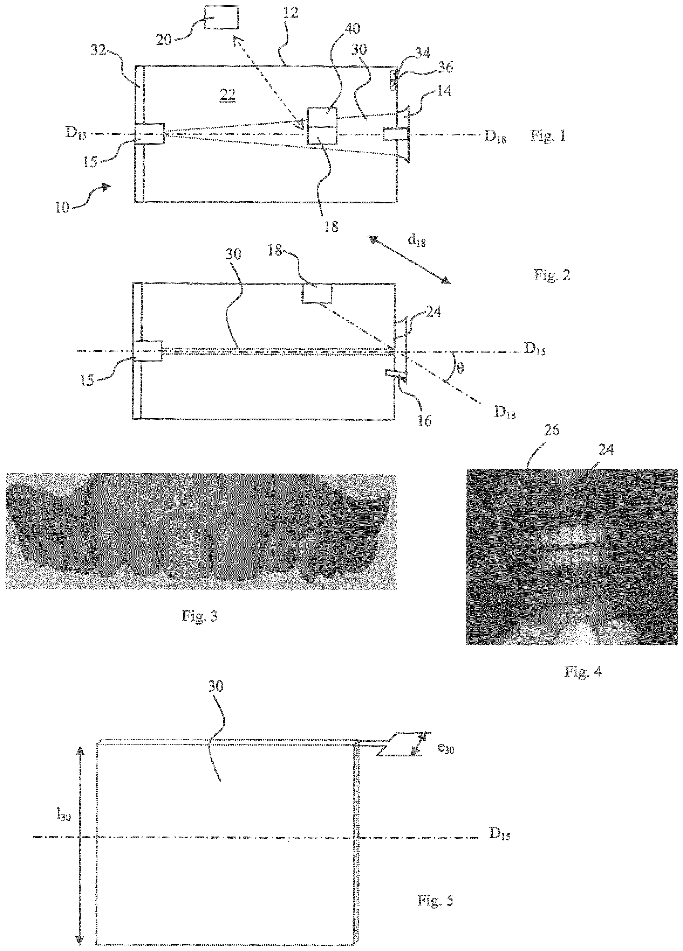

Le dispositif d'acquisition 10 représenté sur la figure 1 comporte un support 12, sous la forme d'un boîtier, de préférence de longueur constante, c'est-à-dire non télescopique, sur lequel sont fixés un écarteur dentaire 14, un projecteur 15, de préférence au moins un miroir 16, un appareil d'acquisition d'images 18, et un module de traitement 20, en communication avec l'appareil d'acquisition d'images.

Le support 12 définit une chambre 22 parallélépipédique d'axe X, qui débouche vers l'extérieur par une ouverture d'écarteur 24.

Le support peut être par exemple en plastique ou en carton.

L'écarteur 14 peut présenter les caractéristiques des écarteurs conventionnels. Il comporte classiquement un rebord 26 s'étendant autour de l'ouverture d'écarteur et agencé de manière que les lèvres du patient puissent y reposer en laissant apparaître les dents du patient à travers ladite ouverture d'écarteur. L'écarteur 14 est de préférence en un matériau biocompatible, par exemple en matière plastique.

L'écarteur 14 peut être venu de matière avec le support 12 ou être fixé, de préférence rigidement, sur le support 12 par tout moyen.

De préférence, l'écarteur est amovible, c'est-à-dire qu'il peut être monté et démonté du support par le patient. Avantageusement, le même support peut donc servir pour plusieurs écarteurs, et en particulier pour plusieurs écarteurs de tailles différentes.

Les moyens de fixation de l'écarteur sur le support peuvent être par exemple des moyens de clipsage, des bandes auto-agrippantes de type Velcro®, des mâchoires de serrage, des vis, des aimants, ou une complémentarité de forme entre le support et l'écarteur.

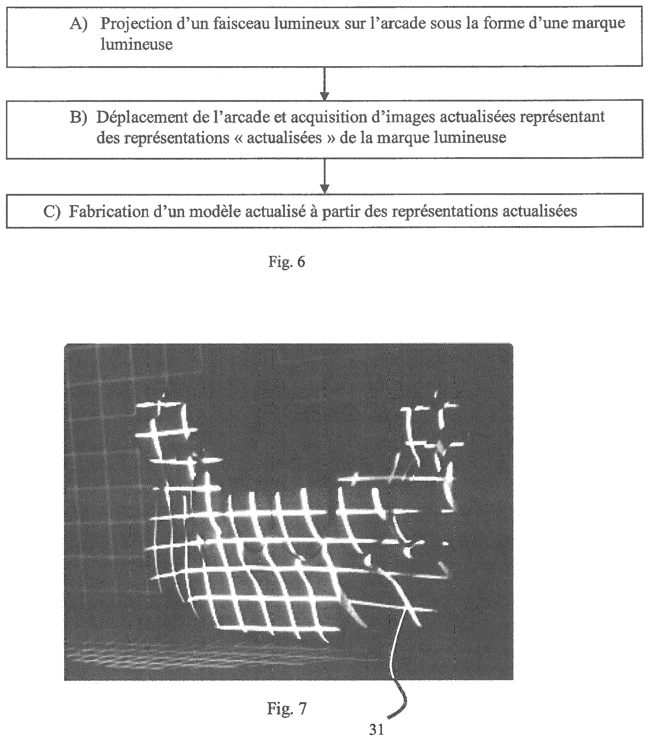

Le projecteur 15 est configuré de manière à projeter un faisceau 30 sous la forme d'une marque lumineuse 31. Sur la figure 7, le faisceau projette ainsi une grille qui est déformée par interaction avec l'arcade dentaire.

La description détaillée qui suit porte sur le cas particulier dans lequel la marque lumineuse est une ligne. L'invention n'est cependant pas limitée à ce cas particulier et, sauf incompatibilité technique, les caractéristiques décrites dans le cadre d'une ligne sont applicables à une autre forme de marque lumineuse.

Lorsque la marque lumineuse est une ligne, le faisceau présente une épaisseur e₃₀ (figure 5), de préférence constante, de préférence inférieure à 1 mm, de préférence inférieure à 0,5 mm, de préférence inférieure à 0,2 mm à sa sortie du projecteur et, de préférence, quand il atteint l'ouverture d'écarteur.

Dans un mode de réalisation, le plan du faisceau forme avec l'axe optique un angle θ supérieur à 10°, de préférence supérieur à 20°, 40° ou 50°, et/ou de préférence inférieur à 80°, de préférence inférieur à 70°. Dans un mode de réalisation, le plan du faisceau forme avec l'axe optique un angle θ supérieur à 1°, et/ou de préférence inférieur à 10°, de préférence inférieur à 5°.

La projection du faisceau est possible directement sur les dents ou par l'intermédiaire d'un ou plusieurs miroirs.

Le faisceau présente de préférence une largeur l₃₀ supérieure à 1 cm, de préférence supérieure à 2 cm quand il atteint l'ouverture d'écarteur.

Dans un plan de coupe transversal à la direction de projection D₁₅, faisant apparaître la largeur et l'épaisseur du faisceau, la section du faisceau peut présenter une forme quelconque. De préférence, le faisceau est sensiblement plan, de préférence vertical, de manière à dessiner sur les dents, à travers l'ouverture d'écarteur 24, au moins une ligne lumineuse déformée, éventuellement interrompue.

Dans un mode de réalisation, la marque lumineuse est variable dans le temps, en particulier lorsque le faisceau est un faisceau de lumière structurée.

De préférence, le faisceau est un faisceau de lumière visible, infra-rouge, ultra-violet ou laser, de préférence non visible. L'utilisation de lumière non visible améliore l'acceptation du dispositif par le patient. En particulier, le patient peut être stressé par une lumière visible, notamment laser.

Un faisceau ultraviolet facilite avantageusement la détection de fissures dans les dents.

Dans un mode de réalisation, le projecteur 15 est configuré de manière à projeter un faisceau 30 dont les caractéristiques, et en particulier la longueur d'onde, varient au cours du temps. L'analyse des images actualisées en est enrichie.

De préférence, le dispositif comporte une source lumineuse 32, de préférence diffuse, de préférence constituée de LED, adaptée pour projeter une lumière facilitant la reconnaissance de la marque lumineuse sur les images acquises, par exemple une lumière de couleur complémentaire à celle de la marque lumineuse. Un éclairage avec une lumière bleue ou cyan peut être bien adapté à une marque lumineuse orange.

La source lumineuse présente de préférence un indice de rendu des couleurs (IRC) supérieur à 80, de préférence supérieur à 90.

De préférence, la chambre 22 ne débouche vers l'extérieur que par l'ouverture d'écarteur.

L'appareil d'acquisition d'images peut être monté amovible sur le support. De préférence, il est fixé de manière non amovible, c'est-à-dire est intégré au support. Avantageusement, il est ainsi possible de fixer définitivement la distance entre l'appareil d'acquisition d'images et l'ouverture d'écarteur, ainsi que l'angle θ entre l'axe optique D₁₈ de l'appareil d'acquisition d'images et le plan du faisceau. La calibration de l'appareil d'acquisition d'images peut être avantageusement plus précise, ce qui permet d'obtenir des images de qualité optimale. Par ailleurs, l'agencement figé de l'appareil d'acquisition d'images et de l'ouverture d'écarteur facilite considérablement la recherche du modèle actualisé.

L'appareil d'acquisition d'images fournit de préférence des images en couleurs, et/ou des images infrarouges. Les images infrarouges permettent avantageusement de faire apparaître les dents avec un excellent contraste.

Dans un mode de réalisation, l'appareil d'acquisition d'images est configuré pour acquérir une image à la demande, c'est-à-dire uniquement lorsqu'un opérateur déclenche cette acquisition.

De préférence, l'appareil d'acquisition d'images est configuré pour acquérir une série comportant plus de 5, 10 ou 20, de préférence plus de 20 images par seconde.

De préférence, l'appareil d'acquisition d'images comporte un capteur multispectral.

L'appareil d'acquisition d'images est de préférence à une distance d₁₈ du centre de l'ouverture d'écarteur inférieure à 20 cm, ou à 10 cm.

Le miroir 16 est de préférence plan. L'orientation du miroir peut être fixe ou variable.

L'appareil d'acquisition d'images est de préférence fixé sur le support dans une position dans laquelle il acquiert une image composée comportant une image directe de l'ouverture d'écarteur et une image de l'ouverture d'écarteur réfléchie par le miroir. Le miroir permet avantageusement de pouvoir observer, sur une même image composée, la marque lumineuse suivant plusieurs directions d'observation. L'image directe et l'image réfléchie, qui représentent la marque lumineuse selon deux directions d'observation différentes, constituent chacune, au sens de l'invention, une image actualisée.

Le miroir 16 permet aussi d'acquérir des images actualisées que l'appareil d'acquisition d'images ne peut acquérir directement, par exemple des vues de dessus.

Dans le mode de réalisation représenté, le miroir 16 est fixé sur l'écarteur. Il peut être également fixé sur le support.

Un miroir, identique ou différent du miroir 16, peut être également utilisé pour détourner le faisceau lumineux, afin que le modèle actualisé puisse représenter des parties de l'arcade difficile, voire impossible à exposer directement au faisceau lumineux.

Le dispositif peut encore comporter une mire colorimétrique 34 et/ou une mire de translucidité 36 fixée(s) sur le support, de préférence dans la chambre 22. Avantageusement, les mires colorimétriques 34 et de translucidité 36 permettent, pour chaque image, de corriger les variations de teinte, et de faciliter l'analyse de l'image actualisée, en particulier pour reconnaître la marque lumineuse.

De préférence, le dispositif comporte encore un télémètre 40 disposé de manière à mesurer une distance entre un point fixe par rapport au support, par exemple un point de l'appareil d'acquisition d'images, et un objet obturant l'ouverture d'écarteur.

De préférence, le dispositif comporte encore une caméra thermique et/ou une sonde de température et/ou un analyseur d'haleine.

Le module de traitement 20 peut être en particulier un calculateur intégré sur le support ou un logiciel chargé dans un ordinateur, un téléphone portable ou une tablette.

Le module de traitement 20 comporte de préférence des moyens de communication, filaires ou non, pour communiquer avec l'appareil d'acquisition d'images et, le cas échéant, le télémètre et/ou la caméra thermique et/ou la sonde de température et/ou l'analyseur d'haleine.

Lorsque le module de traitement 20 n'est pas fixé sur le support, il comporte de préférence des moyens de communication sans fil, par exemple Bluetooth® ou WiFi.

### Procédé

Le procédé est décrit dans le cadre d'une utilisation du dispositif d'acquisition selon l'invention. Il n'est cependant pas limité à ce cadre.

**A l'étape A),** l'opérateur fixe l'écarteur 14 sur le support 12. Le patient, qui peut être également l'opérateur, dispose alors ses lèvres dans les goulottes définies par le rebord de l'écarteur. Comme représenté sur la figure 3, les dents du patient sont alors bien dégagées.

En appuyant sur le déclencheur de l'appareil d'acquisition, l'opérateur provoque l'émission d'un faisceau lumineux 30, de manière à projeter, directement ou par l'intermédiaire d'un ou plusieurs miroirs, une marque lumineuse sur une arcade dentaire du patient.

Dans un mode de réalisation, le faisceau est plan et le plan du faisceau est sensiblement vertical lorsque le patient tient sa tête droite.

En appuyant sur le déclencheur de l'appareil d'acquisition, l'opérateur provoque l'allumage de la source lumineuse 32 et, de préférence, une calibration de l'appareil d'acquisition, de préférence en utilisant des mesures effectuées par le télémètre. Il provoque ensuite l'acquisition d'une série d'images actualisées. De préférence, cette situation est conservée tant que l'opérateur maintient une pression sur le déclencheur.

**A l'étape B),** le patient déplace alors ses dents de manière que le faisceau parcourt l'arcade pendant l'acquisition des images actualisées. Plus précisément, il tourne la tête par rapport au support, en conservant ses lèvres sur l'écarteur, ce qui lui permet de venir exposer au faisceau successivement ses incisives, ses canines, puis ses molaires, d'un côté puis de l'autre.

Si le support comporte un miroir 16, les images acquises peuvent être des images composées chacune d'une image directe et d'au moins une image réfléchie par le miroir.

Les mesures acquises par le télémètre et/ou la caméra thermique et/ou la sonde de température et/ou l'analyseur d'haleine sont transmises au module de traitement 20.

**A l'étape C),** le module de traitement 20 traite les images acquises, suivant des méthodes de traitement conventionnelles, afin d'isoler l'image directe et la ou les images réfléchies, et ainsi de multiplier les images actualisées.

Dans un mode de réalisation, le traitement comporte une opération d'inversion des images réfléchies et/ou une opération de correction des effets de perspective et/ou une opération de correction des couleurs au moyen de la mire colorimétrique.

Ensuite, le module de traitement 20 identifie les projections actualisées sur les images actualisées. L'identification d'une projection actualisée sur une image actualisée peut être réalisée par toute méthode traitement d'images.

Enfin, le module de traitement 20 recherche le modèle actualisé. Les procédés à cet effet ne sont pas limitatifs.

La recherche du modèle actualisé résulte d'une optimisation, de préférence au moyen d'une méthode métaheuristique, et/ou de la mise en œuvre d'un dispositif d'apprentissage profond.

Les méthodes métaheuristiques sont des méthodes d'optimisation connues. La méthode est de préférence choisie dans le groupe formé par
- les algorithmes évolutionnistes, de préférence choisie parmi: les stratégies d'évolution, les algorithmes génétiques, les algorithmes à évolution différentielle, les algorithmes à estimation de distribution, les systèmes immunitaires artificiels, la recomposition de chemin Shuffled Complex Evolution, le recuit simulé, les algorithmes de colonies de fourmis, les algorithmes d'optimisation par essaims particulaires, la recherche avec tabous, et la méthode GRASP ;
- l'algorithme du kangourou,
- la méthode de Fletcher et Powell,
- la méthode du bruitage,
- la tunnelisation stochastique,
- l'escalade de collines à recommencements aléatoires,
- la méthode de l'entropie croisée, et
- les méthodes hybrides entre les méthodes métaheuristiques citées ci-dessus.

L'optimisation procède par itération.

De préférence, on crée un modèle à tester, puis on détermine une distance représentative de la différence entre l'ensemble des projections actualisées et le modèle à tester.

Par exemple, si le faisceau est plan, la projection actualisée de la marque lumineuse sur une image actualisée est formée de la représentation, sur cette image, de la projection d'une ligne, éventuellement fragmentée.

Si le faisceau n'est pas plan, par exemple est constitué d'une grille, la projection actualisée de la marque lumineuse sur une image actualisée est une forme complexe. La figure 7 est un exemple d'image actualisée faisant apparaitre la projection actualisée de la grille sur l'arcade.

Pour évaluer la distance représentative, on détermine d'abord, pour chaque représentation actualisée, une distance élémentaire avec le modèle à tester. A cet effet, on recherche une image de référence optimale, c'est-à-dire une vue du modèle à tester qui permet d'observer une représentation de référence optimale, c'est-à-dire la représentation de référence qui ressemble le plus à la représentation actualisée. On considère alors que l'image de référence optimale présente une concordance maximale avec l'image actualisée.

Ensuite, on évalue la différence entre la projection de référence optimale et la projection actualisée, par exemple en calculant la distance moyenne, en mm ou en nombre de pixels, entre ces deux projections. Cette différence peut constituer la distance élémentaire.

La distance représentative peut être, par exemple, la moyenne des distances élémentaires. Elle fournit une indication sur la concordance du modèle à tester avec l'ensemble des images actualisées de la série.

Le modèle à tester est alors modifié et le cycle reprend avec le nouveau modèle à tester. La modification est de préférence déterminée pour minimiser le nombre de cycles (évaluation de la distance représentative, modification du modèle à tester).

Les cycles sont répétés jusqu'à obtenir une distance représentative inférieure à un seuil d'acceptabilité prédéterminé, de préférence considérée comme minimale.

Le modèle à tester correspondant à cette distance représentative constitue le modèle actualisé.

Pour accélérer l'étape C), le premier modèle à tester est de préférence un modèle d'arcade.

Le premier modèle à tester peut être un modèle de l'arcade du patient, par exemple réalisé au début d'un traitement orthodontique. Le premier modèle à tester peut être un modèle théorique, qui ne représente pas une arcade d'un patient particulier. De préférence, le premier modèle à tester est un modèle d'arcade répondant à des caractéristiques du patient, par exemple un modèle d'arcade typique des patients du même âge et/ou de même sexe et/ou ayant subi le même traitement ou un traitement similaire et/ou ayant le même nombre de dents, aux mêmes emplacements.

Dans un mode de réalisation préféré, le modèle à tester est segmenté, de préférence pour définir un modèle de dent pour chaque dent de l'arcade. La modification du modèle à tester peut avantageusement comprendre des déplacements des modèles de dent et/ou des déformations de ces modèles de dent. La recherche du modèle actualisé en est considérablement accélérée.

Les modifications des modèles à tester successifs sont déterminées pour être réalistes, par exemple pour ne pas conduire à une interpénétration de modèles de dent, ou à des positions ou à des déformations de dent physiologiquement impossibles.

Un dispositif d'apprentissage profond, de préférence un réseau de neurones, est un ensemble d'algorithmes bien connu de l'homme de l'art.

Le réseau de neurones peut être en particulier choisi parmi :
- les réseaux spécialisés dans la classification d'images, appelés « CNN » (« Convolutional neural network »), par exemple
   - AlexNet (2012)
   - ZF Net (2013)
   - VGG Net (2014)
   - GoogleNet (2015)
   - Microsoft ResNet (2015)
   - Caffe: BAIR Reference CaffeNet, BAIR AlexNet
   - Torch:VGG_CNN_S,VGG_CNN_M,VGG_CNN_M_2048,VGG_CNN_M_1 024,VGG_CNN_M_128,VGG_CNN_F,VGG ILSVRC-2014 16-layer,VGG ILSVRC-2014 18-layer,Network-in-Network (Imagenet & CIFAR-10)
   - Google : Inception (V3, V4) ;
- les réseaux spécialisés dans la localisation, et détection d'objets dans une image, les Object Détection Network, par exemple:
   - R-CNN (2013)
   - SSD (Single Shot MultiBox Detector : Object Détection network), Faster R-CNN (Faster Region-based Convolutional Network method : Object Détection network)
   - Faster R-CNN (2015)
   - SSD (2015).

La liste ci-dessus n'est pas limitative.

Classiquement, le dispositif d'apprentissage profond est entrainé par un processus d'apprentissage appelé « *deep learning ».*

Une base d'apprentissage est d'abord constituée. Elle comporte des modèles d'arcades historiques et, pour chaque modèle historique, un ensemble de projections historiques associées.

En présentant, en entrée du dispositif d'apprentissage profond, les modèles historiques et les ensembles de projections historiques correspondant, le dispositif d'apprentissage profond apprend progressivement à reconnaître, à partir d'un ensemble de projections, des motifs, en anglais « *patterns* », et à les associer à des modèles d'arcades.

Après avoir ainsi entrainé le dispositif d'apprentissage profond, on peut lui soumettre l'ensemble des projections actualisées de la série d'images actualisées. Grâce à son entrainement, le dispositif d'apprentissage profond est capable d'y reconnaître des motifs et de déterminer un modèle d'arcade répondant à cet ensemble de projections actualisées, et donc pouvant être considéré comme modèle actualisé.

Bien entendu, les techniques d'optimisation et d'apprentissage profond peuvent être combinées.

Par exemple, on peut d'abord utiliser un dispositif d'apprentissage profond pour créer le premier modèle à tester, puis modifier ce modèle pour l'optimiser.

On peut également utiliser un dispositif d'apprentissage profond pour reconnaître la nature de chaque dent représentée sur les images actualisées (par exemple « incisive » ou « molaire »), puis rechercher, dans une base de modèles de dents, le modèle de dent de même nature qui correspond au mieux à ladite dent. La technique décrite ci-dessus pour rechercher un modèle d'arcade peut être utilisée pour rechercher les modèles de dent.

Après que les modèles de dent ont été identifiés, on peut les agencer pour constituer le premier modèle d'arcade à tester, puis modifier ce premier modèle pour l'optimiser, comme décrit ci-dessus.

D'autres procédés pour fabriquer le modèle actualisé sont encore possibles.

En particulier, l'analyse des projections actualisées peut permettre une évaluation de la distance entre l'appareil d'acquisition d'images et l'arcade dentaire.

Dans un mode de réalisation, on analyse la déformation de la projection actualisée par rapport à sa forme si elle avait été projetée sur un écran perpendiculaire à la direction de projection. Cette analyse permet d'évaluer les reliefs de l'arcade, c'est-à-dire les variations de distance entre le projecteur et l'arcade dans la région dans laquelle la marque lumineuse est projetée. Les algorithmes permettant d'évaluer les variations de distance en fonction de la déformation de la projection actualisée sont connus.

Pour lever d'éventuelles ambiguïtés, par exemple lorsque la marque lumineuse s'étend sur une région comportant des trous, des occlusions, ou des changements de profondeur rapide, le faisceau est de préférence un faisceau de lumière structurée, du type de ceux utilisés par certains scanners 3D, et on peut utiliser notamment la triangulation laser multibande, en anglais *"Multistripe laser Triangulation* (MLT)".

De préférence toujours, le faisceau lumineux est configuré pour projeter un motif non répétitif sur un écran perpendiculaire à la direction de projection, ce qui facilite la levée des ambiguïtés.

L'angle entre un plan perpendiculaire à la direction de projection D₁₅ du faisceau et l'axe optique est de préférence supérieur à 1° et/ou inférieur à 10°, de préférence inférieur à 5°.

L'analyse des projections actualisées peut également comprendre une analyse comparative de projections actualisées d'une même marque lumineuse représentée sur des images actualisées prises en stéréovision, c'est-à-dire prises simultanément par plusieurs appareils d'acquisition d'images présentant des axes optiques différents. De préférence, l'écart angulaire antre les axes optique est supérieur à 1° et/ou inférieur à 10°, de préférence inférieur à 5°.

Pour améliorer l'analyse des projections actualisées, on peut encore évaluer des distances par analyse des images actualisées. En particulier, les dimensions entre deux points remarquables de l'écarteur sont connues, ce qui permet d'évaluer le déplacement relatif de l'appareil d'acquisition d'images par rapport à l'arcade entre deux images actualisées successives.

Le déplacement de l'arcade à travers le faisceau peut résulter du déplacement de l'arcade, le faisceau restant immobile, du déplacement du faisceau, l'arcade restant immobile, ou du déplacement simultané de l'arcade et du faisceau.

Dans un mode de réalisation, le déplacement de l'arcade à travers le faisceau résulte exclusivement du déplacement du faisceau, et ce déplacement est contrôlé. On demande alors au patient de ne pas bouger, et on déplace le faisceau pour qu'il balaie l'arcade à une vitesse contrôlée, par exemple à vitesse constante. Le déplacement contrôlé nécessite cependant de prévoir des moyens d'entraînement du projecteur ou d'un miroir de renvoi du faisceau, par exemple un moteur électrique.

De préférence, le déplacement de l'arcade à travers le faisceau n'est pas contrôlé mécaniquement, notamment parce que le patient peut bouger ses arcades par rapport au faisceau à une vitesse qui n'est pas connue.

Une seule méthode d'analyse des projections actualisées peut donc s'avérer insuffisante. Par exemple, si la marque lumineuse est une ligne verticale et qu'elle n'est observée que par un unique appareil d'acquisition d'images, l'analyse ne permet pas nécessairement de déterminer des informations précises suivant la direction de défilement des dents à travers le faisceau.

De préférence, plusieurs méthodes d'analyse sont donc combinées. De préférence, au moins une des méthodes met en œuvre un réseau de neurones ou une méthode métaheuristique.

A l'issue de l'étape C), on obtient un modèle actualisé qui représente bien l'arcade dentaire ayant fait l'objet de la série d'images actualisées.

Avantageusement, le procédé ne requiert pas la mise en œuvre d'un scanner et peut être mis en œuvre facilement, éventuellement par le patient lui-même, sans avoir à introduire d'objet à l'intérieur de sa bouche.

Comme cela apparaît clairement à présent, un dispositif selon l'invention permet avantageusement de créer un modèle de l'arcade dentaire de manière très rapide, sans avoir recours à un scanner, ni à une personne spécialisée, notamment un dentiste ou un orthodontiste. Le procédé peut être en particulier mis en œuvre par le patient lui-même ou par un de ses proches, n'importe où, et en particulier en dehors d'un cabinet médical, dentaire ou d'orthodontie.

Bien entendu, l'invention n'est pas limitée aux modes de réalisation décrits et représentés, fournis à des fins illustratives seulement.

En particulier, pour faciliter l'analyse, la marque lumineuse peut résulter de la projection de plusieurs lignes ou d'une grille, ou d'un ensemble de points, de préférence non alignés, ou d'une bande. Une bande lumineuse présente avantageusement une largeur déterminée, ce qui facilite la détermination des dimensions. En outre, les bords d'une bande fournissent la même information technique que deux lignes. La largeur de la bande est de préférence supérieure à 0,5 mm, 1 mm, 2 mm, 3 mm et/ou inférieure à 10 mm.

Dans un mode de réalisation, l'orientation du faisceau autour de la direction de projection est variable. Dans un mode de réalisation, elle varie pendant l'acquisition des images actualisées.

En outre, les positions de l'appareil d'acquisition d'images et du projecteur par rapport au plan de l'ouverture d'écarteur peuvent être différentes de celles décrites ci-dessus et représentées. En particulier, la direction de projection D₁₅ n'est pas nécessairement perpendiculaire au plan de l'ouverture d'écarteur.

Dans un mode de réalisation, l'axe optique D₁₈ de l'appareil d'acquisition d'images est perpendiculaire au plan de l'ouverture d'écarteur. Cette situation correspond par exemple au mode de réalisation des figures 1 et 2 dans lequel les positions de l'appareil d'acquisition d'images et du projecteur seraient inversées.

## Revendications

1. Procédé de génération d'un modèle numérique tridimensionnel d'une arcade dentaire d'un patient, au moyen d'un dispositif d'acquisition comportant :
- un support (12) ;
- un écarteur dentaire (14) fixé sur le support et définissant une ouverture d'écarteur (24) ;
- un appareil d'acquisition d'images actualisées (18) fixé sur le support dans une position dans laquelle il observe l'ouverture d'écarteur suivant un axe optique (D₁₈) ;
- un projecteur (15) adapté pour projeter un faisceau lumineux (30) vers l'ouverture d'écarteur, de manière à dessiner, lorsque l'écarteur est porté par le patient, au moins une marque lumineuse sur l'arcade du patient, et
- un module de traitement (20),
ledit procédé comportant les étapes suivantes :
A) projection, au moyen du projecteur (15), d'au moins un faisceau lumineux (30) sur l'arcade, de manière à dessiner au moins une marque lumineuse sur l'arcade ;
B) simultanément à l'étape A), déplacement de l'arcade à travers le faisceau lumineux (30) et acquisition, pendant ledit déplacement, d'une série de dites images actualisées de ladite arcade faisant chacune apparaître une représentation de la marque lumineuse projetée, ou « projection actualisée », « simultanément à l'étape A) », signifiant que la série d'images actualisées est acquise alors que le faisceau lumineux est projeté, ladite projection pouvant cependant commencer avant et se prolonger après l'acquisition des images actualisées ;
C) identification, par le module de traitement (20), de ladite projection actualisée sur chaque image actualisée, puis fabrication, par le module de traitement (20), d'un modèle numérique tridimensionnel, dit « modèle actualisé », présentant une concordance maximale avec l'ensemble des projections actualisées, le modèle actualisé étant recherché au moyen d'une méthode d'optimisation et/ou d'un dispositif d'apprentissage profond.

2. Procédé selon la revendication précédente, dans lequel, à l'étape C), on détermine le modèle actualisé comme étant le modèle à tester obtenu à la fin du cycle d'étapes a) à c) suivant :
a) création d'un modèle à tester, puis
b) détermination d'une distance représentative de la différence entre l'ensemble des projections actualisées et le modèle à tester, puis,
c) si ladite distance représentative dépasse un seuil d'acceptabilité prédéterminé, modification du modèle à tester et reprise à l'étape a),
la distance représentative étant déterminée à partir de distances élémentaires, chaque distance élémentaire étant déterminée, pour une projection actualisée respective, par une évaluation de la différence entre ladite projection actualisée et une projection de référence optimale,
une projection de référence étant une représentation, sur une image de référence représentant une vue du modèle à tester, d'une marque lumineuse virtuelle résultant de la projection, sur le modèle à tester, d'un faisceau lumineux virtuel de même forme que le faisceau lumineux projeté sur l'arcade à l'étape A),
l'image de référence optimale étant l'image de référence faisant apparaître la projection de référence qui présente une distance minimale avec la projection actualisée.

3. Procédé selon la revendication immédiatement précédente, dans lequel le modèle à tester est segmenté de manière à définir des modèles de dent et, à l'étape c), la modification du modèle à tester comprend des déplacements des modèles de dent et/ou des déformations de ces modèles de dent.

4. Procédé selon l'une quelconque des revendications précédentes, dans lequel, à l'étape C), pour fabriquer le modèle actualisé, on modifie un modèle défini en fonction de caractéristiques du patient.

5. Procédé selon l'une quelconque des revendications précédentes, dans lequel à l'étape C), on met en œuvre au moins deux méthodes parmi les méthodes d'optimisation, les méthodes d'intelligence artificielle, les méthodes d'évaluation de dimensions par stéréovision, les méthodes d'évaluation de dimensions par analyse de la forme de la marque lumineuse et les méthodes d'évaluation de dimensions par analyse de la distance entre des points remarquable des images actualisées.

6. Procédé selon l'une quelconque des revendications précédentes, dans lequel à l'étape A), on projette un faisceau de lumière structurée.

7. Procédé selon l'une quelconque des revendications précédentes, dans lequel à l'étape B), le patient porte l'écarteur dentaire immobilisé par rapport à l'appareil d'acquisition desdites images actualisées, et déplace ladite arcade par rapport audit appareil d'acquisition d'images.

8. Procédé selon l'une quelconque des revendications précédentes, dans lequel le dispositif d'acquisition comporte un miroir (16) configuré pour réfléchir une image vers l'appareil d'acquisition d'images et/ou un miroir configuré pour réfléchir le faisceau lumineux, et/ou un capteur multispectral et/ou une mire colorimétrique (34) et/ou une mire de translucidité (36) et/ou une caméra thermique et/ou une sonde de température et/ou un analyseur d'haleine et/ou un télémètre, en communication avec l'appareil d'acquisition d'images et disposé de manière à mesurer une distance entre un point du support et un objet obturant l'ouverture d'écarteur.

9. Procédé selon l'une quelconque des revendications précédentes, dans lequel l'appareil d'acquisition d'images actualisées (18) et/ou le module de traitement (20) sont intégrés sur le support.

10. Procédé selon l'une quelconque des revendications précédentes, dans lequel le projecteur est configuré pour projeter un faisceau non visible à l'œil nu.

11. Procédé selon l'une quelconque des revendications précédentes, dans lequel le projecteur est configuré pour projeter un faisceau ultraviolet.

12. Procédé selon l'une quelconque des revendications précédentes, dans lequel le projecteur (15) est configuré de manière à faire varier la longueur d'onde et/ou la forme du faisceau (30) au cours du temps.

13. Procédé selon l'une quelconque des revendications précédentes, dans lequel la marque lumineuse est un point, une ligne, une bande ou un ensemble de points et/ou de lignes et/ou de bandes.

14. Procédé selon l'une quelconque des revendications précédentes, dans lequel la marque lumineuse est une ligne et le faisceau présente une épaisseur (e₃₀), de préférence constante, inférieure à 1 mm à la sortie du projecteur et quand il atteint l'ouverture d'écarteur.

15. Procédé selon la revendication immédiatement précédente, dans lequel le plan du faisceau forme avec l'axe optique un angle θ supérieur à 10° et inférieur à 80°.

## Patentansprüche

1. Verfahren zur Erzeugung eines dreidimensionalen numerischen Modells einer Zahnreihe eines Patienten mittels einer Erfassungsvorrichtung, welche umfasst:
- einen Träger (12);
- einen Wangenhalter (14), der auf dem Träger befestigt ist und eine Wangenhalteröffnung (24) definiert;
- eine Einrichtung zur Erfassung aktualisierter Bilder (18), die auf dem Halter in einer Position befestigt ist, in welcher sie die Wangenhalteröffnung entlang einer optischen Achse (D₁₈) betrachtet;
- einen Strahler (15), der dafür eingerichtet ist, ein Lichtbündel (30) in Richtung der Wangenhalteröffnung auszusenden, um so, wenn der Wangenhalter von dem Patienten getragen wird, wenigstens eine Lichtmarke auf der Zahnreihe des Patienten zu zeichnen, und
- ein Verarbeitungsmodul (20),
wobei das Verfahren die folgenden Schritte umfasst:
A) Projektion, mittels des Strahlers (15), mindestens eines Lichtbündels (30) auf die Zahnreihe, um so mindestens eine Lichtmarke auf der Zahnreihe zu zeichnen;
B) gleichzeitig mit Schritt A), Bewegung der Zahnreihe durch das Lichtbündel (30) und Erfassung, während der Bewegung, einer Reihe von sogenannten aktualisierten Bildern der Zahnreihe, in denen jeweils eine Darstellung der projizierten Lichtmarke, oder "aktualisierte Projektion", erscheint, wobei "gleichzeitig mit Schritt A)" bedeutet, dass die Reihe von aktualisierten Bildern erfasst wird, während das Lichtbündel projiziert wird, wobei diese Projektion jedoch vor der Erfassung der aktualisierten Bilder beginnen und sich nach dieser fortsetzen kann;
C) Identifikation, durch das Verarbeitungsmodul (20), der aktualisierten Projektion auf jedem aktualisierten Bild, danach Erstellung, durch das Verarbeitungsmodul (20), eines dreidimensionalen numerischen Modells, "aktualisiertes Modell" genannt, das eine maximale Übereinstimmung mit der Menge der aktualisierten Projektionen aufweist, wobei das aktualisierte Modell mittels eines Optimierungsverfahrens und/oder einer auf Deep Learning basierenden Vorrichtung gesucht wird.

2. Verfahren nach dem vorhergehenden Anspruch, wobei in Schritt C) das aktualisierte Modell als das zu testende Modell bestimmt wird, das am Ende des folgenden Zyklus von Schritten a) bis c) erhalten wird:
a) Erzeugung eines zu testenden Modells, danach
b) Bestimmung eines Abstands, der für den Unterschied zwischen der Menge der aktualisierten Projektionen und dem zu testenden Modell repräsentativ ist, danach
c) falls der repräsentative Abstand einen vorbestimmten Akzeptanzschwellenwert überschreitet, Änderung des zu testenden Modells und Beginn wieder bei Schritt a),
wobei der repräsentative Abstand aus elementaren Abständen bestimmt wird, wobei jeder elementare Abstand, für eine jeweilige aktualisierte Projektion, durch eine Bewertung des Unterschieds zwischen dieser aktualisierten Projektion und einer optimalen Referenzprojektion bestimmt wird,
wobei eine Referenzprojektion eine Darstellung, auf einem eine Ansicht des zu testenden Modells darstellenden Referenzbild, einer virtuellen Lichtmarke ist, die aus der Projektion, auf das zu testende Modell, eines virtuellen Lichtbündels resultiert, das dieselbe Form wie das in Schritt A) auf die Zahnreihe projizierte Lichtbündel hat, wobei das optimale Referenzbild das Referenzbild ist, in dem die Referenzprojektion erscheint, welche einen minimalen Abstand von der aktualisierten Projektion aufweist.

3. Verfahren nachdem unmittelbar vorhergehenden Anspruch, wobei das zu testende Modell so segmentiert wird, dass Zahnmodelle definiert werden und in Schritt c) die Änderung des zu testenden Modells Bewegungen der Zahnmodelle und/oder Verformungen dieser Zahnmodelle umfasst.

4. Verfahren nach einem der vorhergehenden Ansprüche, wobei in Schritt C), um das aktualisierte Modell zu erstellen, ein in Abhängigkeit von Merkmalen des Patienten definiertes Modell geändert wird.

5. Verfahren nach einem der vorhergehenden Ansprüche, wobei in Schritt C) mindestens zwei Verfahren von den Optimierungsverfahren, den Verfahren der künstlichen Intelligenz, den Verfahren zur Bestimmung von Abmessungen durch Stereovision, den Verfahren zur Bestimmung von Abmessungen durch Analyse der Form der Lichtmarke und den Verfahren zur Bestimmung von Abmessungen durch Analyse des Abstands zwischen bemerkenswerten Punkten der aktualisierten Bilder durchgeführt werden.

6. Verfahren nach einem der vorhergehenden Ansprüche, wobei in Schritt A) ein strukturiertes Lichtbündel projiziert wird.

7. Verfahren nach einem der vorhergehenden Ansprüche, wobei in Schritt B) der Patient den Wangenhalter trägt, der in Bezug auf die Einrichtung zur Erfassung der aktualisierten Bilder fixiert ist, und die Zahnreihe in Bezug auf die Einrichtung zur Erfassung von Bildern bewegt.

8. Verfahren nach einem der vorhergehenden Ansprüche, wobei die Erfassungsvorrichtung einen Spiegel (16), der dafür ausgelegt ist, ein Bild zu der Einrichtung zur Erfassung von Bildern zu reflektieren, und/oder einen Spiegel, der dafür ausgelegt ist, das Lichtbündel zu reflektieren, und/oder einen Multispektralsensor und/oder ein Farbtestbild (34) und/oder ein Transluzenz-Testbild (36) und/oder eine Wärmebildkamera und/oder eine Temperatursonde und/oder einen Atemanalysator und/oder ein Telemeter, das mit der Einrichtung zur Erfassung von Bildern in Verbindung steht und so angeordnet ist, dass es einen Abstand zwischen einem Punkt des Trägers und einem die Wangenhalteröffnung verschließenden Objekt misst, umfasst.

9. Verfahren nach einem der vorhergehenden Ansprüche, wobei die Einrichtung zur Erfassung aktualisierter Bilder (18) und/oder das Verarbeitungsmodul (20) in den Träger integriert sind.

10. Verfahren nach einem der vorhergehenden Ansprüche, wobei der Strahler dafür ausgelegt ist, ein mit bloßem Auge nicht sichtbares Lichtbündel auszusenden.

11. Verfahren nach einem der vorhergehenden Ansprüche, wobei der Strahler dafür ausgelegt ist, ein ultraviolettes Lichtbündel auszusenden.

12. Verfahren nach einem der vorhergehenden Ansprüche, wobei der Strahler (15) dafür ausgelegt ist, ein Variieren der Wellenlänge und/oder der Form des Lichtbündels (30) im Laufe der Zeit zu bewirken.

13. Verfahren nach einem der vorhergehenden Ansprüche, wobei die Lichtmarke ein Punkt, eine Linie, ein Streifen oder eine Menge von Punkten und/oder Linien und/oder Streifen ist.

14. Verfahren nach einem der vorhergehenden Ansprüche, wobei die Lichtmarke eine Linie ist und das Lichtbündel eine vorzugsweise konstante Dicke (e₃₀) aufweist, die am Ausgang des Strahlers und beim Erreichen der Wangenhalteröffnung kleiner als 1 mm ist.

15. Verfahren nach dem vorhergehenden Anspruch, wobei die Ebene des Lichtbündels mit der optischen Achse einen Winkel θ bildet, der größer als 10° und kleiner als 80° ist.

## Claims

1. A method for generating a three-dimensional digital model of a dental arch of a patient, by means of an acquisition device comprising:
- a support (12);
- a dental retractor (14) fixed to the support and defining a retractor aperture (24);
- an updated image acquisition unit (18) fixed to the support in a position in which it observes the retractor aperture along an optical axis (Dis);
- a projector (15) suitable for projecting a light beam (30) toward the retractor aperture, so as to draw, when the retractor is worn by the patient, at least one light mark on the arch of the patient, and
- a processing module (20),
said method comprising the following steps:
A) projection, by means of the projector (15), of at least one light beam (30) onto the arch, so as to draw at least one light mark on the arch;
B) simultaneously with the step A), displacement of the arch across the light beam (30) and acquisition, during said displacement, of a series of said updated images of said arch each showing a representation of the projected light mark, or "updated projection", "simultaneously with the step A)" meaning that the series of updated images is acquired while the beam is projected, whereas said projection can however begin before and be prolonged after the acquisition of the updated images;
C) identification, by the processing module (20), of said updated projection on each updated image, then production, by the processing module (20), of a three-dimensional digital model, called "updated model", exhibiting a best fit with all the updated projections, the updated model being sought by means of an optimization method and/or a deep learning device.

2. The method as claimed in the preceding claim, wherein, in the step C), the updated model is determined to be the model to be tested obtained at the end of the following cycle of steps a) to c):
a) creation of a model to be tested, then
b) determination of a distance representative of the difference between all the updated projections and the model to be tested, then,
c) if said representative distance exceeds a predetermined acceptability threshold, modification of the model to be tested and return to the step a),
the representative distance being determined from elementary distances, each elementary distance being determined, for a respective updated projection, by an evaluation of the difference between said updated projection and an optimal reference projection,
a reference projection being a representation, on a reference image representing a view of the model to be tested, of a virtual light mark resulting from the projection, on the model to be tested, of a virtual light beam of the same form as the light beam projected onto the arch in the step A),
the optimal reference image being the reference image showing the reference projection which exhibits a minimum distance with the updated projection.

3. The method as claimed in the immediately preceding claim, wherein the model to be tested is segmented so as to define tooth models and, in the step c), the modification of the model to be tested comprises displacements of the tooth models and/or deformations of these tooth models.

4. The method as claimed in any one of the preceding claims, wherein, in the step C), to produce the updated model, a model defined according characteristics of the patient is modified.

5. The method as claimed in any one of the preceding claims, wherein, in the step C), at least two methods are implemented from among the optimization methods, the artificial intelligence methods, the methods for evaluating dimensions by stereovision, the methods for evaluating dimensions by analysis of the form of the light mark and the methods for evaluating dimensions by analysis of the distance between noteworthy points of the updated images.

6. The method as claimed in any one of the preceding claims, wherein, in the step A), a structured light beam is projected.

7. The method as claimed in any one of the preceding claims, wherein, in the step B), the patient wears the dental retractor immobilized with respect to the unit for acquiring said updated images, and displaces said arch with respect to said image acquisition unit.

8. The method as claimed in any one of the preceding claims, wherein the acquisition device comprises a mirror (16) configured to reflect an image toward the image acquisition unit and/or a mirror configured to reflect the light beam, and/or a multispectral sensor and/or a colorimetric pattern (34) and/or a translucency pattern (36) and/or a thermal camera and/or a temperature probe and/or a halitus analyzer and/or a distance meter, in communication with the image acquisition unit and disposed so as to measure a distance between a point of the support and an object closing the retractor aperture.

9. The method as claimed in any one of the preceding claims, wherein the updated image acquisition unit (18) and/or the processing module (20) are incorporated on the support.

10. The method as claimed in any one of the preceding claims, wherein the projector is configured to project a beam that is not visible to the naked eye.

11. The method as claimed in any one of the preceding claims, wherein the projector is configured to project an ultraviolet beam.

12. The method as claimed in any one of the preceding claims, wherein the projector (15) is configured so as to vary the wavelength and/or the form of the beam (30) over time.

13. The method as claimed in any one of the preceding claims, wherein the light mark is a dot, a line, a strip or a set of dots and/or of lines and/or of strips.

14. The method as claimed in any one of the preceding claims, wherein the light mark is a line and the beam has a thickness (e₃₀), preferably constant, less than 1 mm at the output of the projector and when it reaches the retractor aperture.

15. The method as claimed in the immediately preceding claim, wherein the plane of the beam forms, with the optical axis, an angle θ greater than 10° and less than 80°.
